(19) **Europäisches Patentamt**
**European Patent Office**
**Office européen des brevets**

(11) **EP 3 981 381 B1**

(12) **EUROPEAN PATENT SPECIFICATION**

(45) Date of publication and mention
of the grant of the patent:
**06.11.2024 Bulletin 2024/45**

(21) Application number: **20818342.6**

(22) Date of filing: **04.06.2020**

(51) International Patent Classification (IPC):
***A61K 8/19*** (2006.01)      ***A61K 8/72*** (2006.01)
***A61K 8/81*** (2006.01)      ***A61Q 17/00*** (2006.01)

(52) Cooperative Patent Classification (CPC):
(C-Sets available)
**C01B 33/18; A61K 8/0241; A61K 8/25;**
**A61K 8/8117; A61K 8/8158; A61K 8/8176;**
**A61Q 17/00; C08F 271/02; C08L 25/06;**
**C08L 33/26; C08L 39/06; C08L 51/003;**
A61K 2800/412; A61K 2800/624; A61Q 1/02

(Cont.)

(86) International application number:
**PCT/JP2020/022175**

(87) International publication number:
**WO 2020/246555 (10.12.2020 Gazette 2020/50)**

(54) **PARTICLE-CONTAINING COMPOSITION**

PARTIKELHALTIGE ZUSAMMENSETZUNG

COMPOSITION CONTENANT DES PARTICULES

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **06.06.2019 JP 2019106324**

(43) Date of publication of application:
**13.04.2022 Bulletin 2022/15**

(73) Proprietor: **Kao Corporation**
**Chuo-ku**
**Tokyo 103-8210 (JP)**

(72) Inventors:
• **TAKIZAWA, Hiroyuki**
**Tokyo 131-8501 (JP)**
• **ASANO, Moeko**
**Wakayama-shi, Wakayama 640-8580 (JP)**
• **FUJII, Tomoya**
**Tokyo 131-8501 (JP)**

(74) Representative: **Hoffmann Eitle**
**Patent- und Rechtsanwälte PartmbB**
**Arabellastraße 30**
**81925 München (DE)**

(56) References cited:
**WO-A1-2008/123284      JP-A- 2005 336 056**
**JP-A- 2012 250 917      JP-A- 2013 107 887**
**JP-A- 2017 105 825      JP-A- 2018 535 970**
**US-A- 4 205 997      US-A1- 2005 074 474**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(52) Cooperative Patent Classification (CPC): (Cont.)

C-Sets
**C08F 271/02, C08F 212/08;**
**C08L 25/06, C08L 39/06, C08K 3/36;**
**C08L 33/06, C08L 39/06, C08K 3/36;**
**C08L 51/003, C08K 3/36**

**Description**

FIELD OF THE INVENTION

The present invention relates to a particle-containing composition, a method for producing the particle-containing composition, and a method for suppressing adhesion of air harmful substances using the particle-containing composition.

BACKGROUND OF THE INVENTION

Inorganic particles exhibit many functions, and therefore, applications thereof to a wide range of industrial fields have been investigated.

For example, there is known a technique for making skin unevenness less noticeable by utilizing optical properties of inorganic particles. For the purpose of providing a cosmetic material having masking performance, JP 2012-250917 (PTL 1) describes a cosmetic material produced by blending a surface-coated powder prepared by coating the surfaces of at least one kind of powdery particle selected from a metal oxide, a metal hydroxide and/or a composite containing them with a specific copolymer, and spherical particles having an average particle diameter as primary particles of 25 to 35 $\mu$m and an oily agent.

In addition, also known is a technique of utilizing adsorption performance of fine pores of an inorganic porous material. For example, WO2014/136993 (PTL 2) describes a skin-care cosmetic material containing a specific amount of magnesium metasilicate aluminate and a specific amount of a UV protectant, as a skin-care cosmetic material capable of protecting skin from external stimulations such as air pollutants.

JP 2012 250917 A describes a cosmetic that is formulated with (A) 1-78 mass% of surface-treated powder obtained by coating the surface of one or more kinds of powder particles, selected from a metal oxide, metal hydroxide and/or a composite thereof, with methylsilanol tri(coconut oil fatty acid PEG-8 glyceryl), coconut oil fatty acid PEG-7 glyceryl, and dimethyldiallylammonium chloride/acrylamide copolymer; (B) 2-50 mass% of a spherical particle with 25-35 $\mu$m average particle size of primary particles; and (C) 20-80 mass% of an oil solution.

US 2005/074474 A1 describes a topical composition comprising: (1) a porous spherical disintegrative silica impregnated with a water-insoluble skin benefit agent, wherein: (a) the porous spherical disintegrative silica has an average volume particle size of from about 3 $\mu$m to about 20 $\mu$m, a maximum particle size of no more than about 50 $\mu$m, and a pore volume of from about 1.5 cm$^3$/g to about 3.0 cm$^3$/g; and provides a certain dynamic viscoelasticity when sheared; (b) the water-in-soluble skin benefit agent having a solubility in water of less than about 0.1 g/1 at 25° C and having a molecular weight of no more than about 5,000, selected from the group consisting of liquid water-insoluble skin benefit agents, solid water-insoluble skin benefit agents which dissolve in liquid water-insoluble skin benefit agents, solid water-insoluble skin benefit agents which dissolve in emollients and/or volatile solvents, and mixtures thereof; and (2) a suitable carrier.

US 4 205 997 A describes powdery pearlescent pigment compositions comprising individual particles of a flaky pearlescent pigment coated with 1 to 50 weight percent of a solid polymer, optionally containing wetting agents, fillers, additional coloring agents, UV-absorbers or scenting agents, produced by combining a suspension of a flaky pearlescent pigment with a solution of a polymer, possibly containing other substances, and subsequently depositing polymer on the pigment flakes.

SUMMARY OF THE INVENTION

The present invention relates to a particle-containing composition that contains inorganic particles (A) and particles (B) other than the particles (A), wherein:

the inorganic particles (A) are at least one selected from the group consisting of the following inorganic particles (A1) and inorganic particles (A2),
the particles (B) are particles having an average particle diameter $D_B$ of 1 $\mu$m or more and 50 $\mu$m or less, and coated on the surfaces thereof with a polymer (C) having an amide bond or an amide group in the side chain,

Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less,

Inorganic particles (A2): inorganic particles having an average particle diameter $D_{A2}$ of less than 1 $\mu$m, and having an average primary particle diameter $d_{A2}$ of 10 nm or more and 20 nm or less,

the inorganic particles (A) are particles containing silica,

the mass ratio of particles (B) to inorganic particles (A), [(B)/(A)], is 0.1 or more and 11 or less, and
the blending amount of the polymer (C) relative to the particles (B) is 7% by mass or more and 35% by mass or less.

BRIEF DESCRIPTION OF THE DRAWING

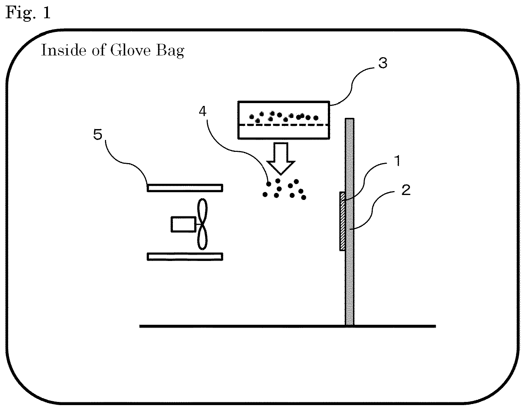

Fig. 1 is an outline side view showing an evaluation method for an adhesion suppressing effect.

DETAILED DESCRIPTION OF THE INVENTION

Recently, air harmful substances floating in air such as pollens of cedar and Japanese cypress, air pollutants such as smoke and dust, and yellow sand have become problematic because they cause various health harm to the human body. Among air harmful substances, particulate matters having a diameter of 2.5 $\mu$m or less, called PM 2.5, are composed of a carbon material, a sulfate, a nitrate, an ammonium salt, etc., as constituent components, and it is known that PM 2.5 and yellow sand cause diseases of circulatory system and respiratory systems by inhalation. In addition, it is pointed out that PM 2.5 as well as pollens and yellow sand adhere to or penetrate through skin to cause skin troubles. For example, Nature Chemistry, 3, 291-295 (2011) by Shiraiwa, et al. discloses an academic report relating to PM 2.5 that causes damage to skin. Consequently, in order to suppress health harm due to exposure to air harmful substances, it is required to suppress adhesion of air harmful substances to various solid surfaces around the body such as skin, hair and clothing.

However, the technique of PTL 1 is directed to a problem of skin unevenness masking performance, and the adhesion suppressing effect is not sufficient. The technique of PTL 2 is to adsorb air harmful substances by the adsorption performance of magnesium metasilicate aluminate to thereby prevent adhesion of air harmful substances to skin, and the adhesion suppressing effect is not sufficient. In addition, inorganic particles in the form of a powder give a squeaky feel, and especially after application to skin, the feel thereof is often poor. Consequently, a good feel after application to a solid surface is desired.

Further, in the technique of PTL 2, the cosmetic material applied to skin drops off from the skin surface by rubbing with hands or clothes, and therefore it has been found that there are some other problems in that the adhesion suppressing effect after rubbing is reduced and the feel is deteriorated.

The present invention relates to a particle-containing composition having a high adhesion suppressing effect against air harmful substances and capable of giving an excellent feel after application and, even after rubbing, capable of maintaining the high adhesion suppressing effect and the good feel, and to a method for producing the particle-containing composition, and a method for suppressing adhesion of air harmful substances using the particle-containing composition.

The present inventors have noted that a particle-containing composition that contains inorganic particles and particles other than the inorganic particles, in which the inorganic particles are at least one selected from the group consisting of easily disintegrable inorganic particles (A1) having an average particle diameter and a surface roughness Ra each falling within a predetermined range, and inorganic particles (A2) having an average particle diameter and an average primary particle diameter each falling within a predetermined range, can enhance the adhesion suppressing effect against air harmful substances when the particle-containing composition is applied to the surface of a solid, while, on the other hand, the other particles have an average particle diameter falling within a specific range and are coated with a polymer having a hydrogen bond-accepting bond or a hydrogen bond-accepting functional group, can enhance the feel after application to the surface of a solid without reducing the adhesion suppressing effect, and have found out a possibility of providing a particle-containing composition having a high adhesion suppressing effect against air harmful substances and capable of giving an excellent feel after application and, even after rubbing, capable of maintaining the high adhesion suppressing effect and the good feel, and also a method for producing the particle-containing composition, and a method for suppressing adhesion of air harmful substances using the particle-containing composition.

Specifically, the present invention relates to the following aspects [1] to [3].

[1] A particle-containing composition that contains inorganic particles (A) and particles (B) other than the particles (A), wherein:

the inorganic particles (A) are at least one selected from the group consisting of the following inorganic particles (A1) and inorganic particles (A2),
the particles (B) are particles having an average particle diameter $D_B$ of 1 $\mu$m or more and 50 $\mu$m or less, and coated on the surfaces thereof with a polymer (C) having an amide bond or an amide group in the side chain,

Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less,

Inorganic particles (A2): inorganic particles having an average particle diameter $D_{A2}$ of less than 1 $\mu$m, and having an average primary particle diameter $d_{A2}$ of 10 nm or more and 20 nm or less,

the inorganic particles (A) are particles containing silica,

the mass ratio of particles (B) to inorganic particles (A), [(B)/(A)], is 0.1 or more and 11 or less, and

the blending amount of the polymer (C) relative to the particles (B) is 7% by mass or more and 35% by mass or less.

[2] A method for producing a particle-containing composition of the above aspect [1] wherein the particles (B) are particles prepared by coating the surfaces of particles (B') with the polymer (C), the method including the following step 1 and step 2,

Step 1: a step of mixing particles (B') and the polymer (C) to give the particles (B),

Step 2: a step of mixing the particles (B) obtained in the step 1 with the inorganic particles (A) to give a particle-containing composition.

[3] A method for suppressing adhesion of air harmful substances, including applying a particle-containing composition of the above aspect [1] to the surface of a solid to suppress adhesion of air harmful substances to the solid surface.

According to the present invention, there can be provided a particle-containing composition having a high adhesion suppressing effect against air harmful substances and capable of giving an excellent feel after application and, even after rubbing, capable of maintaining the high adhesion suppressing effect and the good feel, as well as a method for producing the particle-containing composition, and a method for suppressing adhesion of air harmful substances using the particle-containing composition.

[Particle-Containing Composition]

The particle-containing composition of the present invention is a particle-containing composition that contains inorganic particles (A) and particles (B) other than the particles (A), wherein the inorganic particles (A) are at least one selected from the group consisting of the following inorganic particles (A1) and inorganic particles (A2), the particles (B) are particles having an average particle diameter $D_B$ of 1 $\mu$m or more and 50 $\mu$m or less, and coated on the surfaces thereof with a polymer (C) having an amide bond or an amide group in the side chain,

Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less,

Inorganic particles (A2): inorganic particles having an average particle diameter $D_{A2}$ of less than 1 $\mu$m, and having an average primary particle diameter $d_{A2}$ of 10 nm or more and 20 nm or less,

the inorganic particles (A) are particles containing silica,

the mass ratio of particles (B) to inorganic particles (A), [(B)/(A)], is 0.1 or more and 11 or less, and

the blending amount of the polymer (C) relative to the particles (B) is 7% by mass or more and 35% by mass or less.

In the present invention, "air harmful substance" mean harmful substance (including PM 2.5) floating in air, such as pollens of cedar and Japanese cypress; air pollutants such as particles containing soot and smoke containing such as sulfur oxides, soot and dust, and nitrogen oxides, powder dust, motor exhaust, hazardous air pollutants such as benzene, trichloroethylene and tetrachloroethylene, and volatile organic compounds (VOC), etc.; and yellow sand.

The particle-containing composition of the present invention has a high adhesion suppressing effect against air harmful substances, especially fine particulate air harmful substances and is excellent in the feel after application of the composition to the surface of a solid, and further can maintain the high adhesion suppressing effect and the good feel even after rubbing. Though not clear, the reason may be considered to be as follows.

The particle-containing composition of the present invention contains, as the inorganic particles (A), at least one kind of particles selected from the group consisting of easily disintegrable inorganic particles (A1) having an average particle diameter and a surface roughness Ra each falling within a specific range, and inorganic particles (A2) having an average particle diameter and an average primary particle diameter each falling within a specific range, and therefore when applied to the surface of a solid, the composition can form local nano-size irregularities on the solid surface. With that, when the surface is brought into contact with air harmful substances, it is considered that the contact area can reduce to effectively suppress adhesion of air harmful substances thereto. Further, the particle-containing composition of the present invention contains particles (B) other than the inorganic particles (A), which have an average particle diameter falling within a specific range and which are coated with a polymer (C) having an amide bond or an amide group in the side chain, and therefore, it is considered that the composition can improve the feel thereof after application to the surface of a solid without reducing the adhesion suppressing effect.

In the case where the coating film formed of the particle-containing composition on the surface of a solid is rubbed, owing to the hydrogen-bonding interaction between the polar surface derived from a hydroxy group of the surfaces of the inorganic particles (A) and the amide bond or amide group of the polymer (C) existing on the surfaces of the particles (B), the inorganic particles (A1) or the inorganic particles (A2) disintegrated by rubbing adhere to the surfaces of the particles (B) to form composite particles. The composite particles are prevented from dropping down from the surface of the solid owing to the high adsorption performance of the polymer (C) to the solid surface in addition to the adhesion suppressing effect by the formation of the nano-size irregularities owing to contribution of the inorganic particles (A), and consequently, it is considered that, even after rubbing, the composition can still exhibit the effect of maintaining the high adhesion suppressing effect and the good feel.

<Inorganic Particles (A)>

The inorganic particles (A) are, from the viewpoint of improving the adhesion suppressing effect and from the viewpoint of maintaining the excellent adhesion suppressing effect after rubbing, at least one selected from the group consisting of the following inorganic particles (A1) and inorganic particles (A2).

Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less.

Inorganic particles (A2): inorganic particles having an average particle diameter $D_{A2}$ of less than 1 $\mu$m, and having an average primary particle diameter $d_{A2}$ of 10 nm or more and 20 nm or less.

Among these, the inorganic particles (A) are, from the viewpoint of improving the adhesion suppressing effect and from the viewpoint of maintaining the excellent adhesion suppressing effect after rubbing, particles containing silica.

The inorganic particles (A) may not be surface-treated or may be surface-treated.

The content of the inorganic substance in the inorganic particles (A) is preferably 60% by mass or more, more preferably 70% by mass or more, and is preferably 100% by mass or less.

In the case where the inorganic particles (A) are surface-treated ones, the mass, the adhering amount, the average particle diameter $D_{A1}$ or $D_{A2}$ to be mentioned below, the average primary particle diameter $d_{A1}$ or $d_{A2}$, and the surface roughness Ra of the inorganic particles (A) mean the mass, the coating amount (that is, the adhering amount), the average particle diameter $D_{A1}$ or $D_{A2}$, the average primary particle diameter $d_{A1}$ or $d_{A2}$, and the surface roughness Ra thereof inclusive of the surface treatment agent.

[Inorganic Particles (A1)]

The inorganic particles (A1) are easily disintegrable secondary inorganic particles having an average particle diameter $D_{A1}$ of 1 $\mu$m or more and 50 $\mu$m or less, and having a surface roughness Ra of 10 nm or more and 50 nm or less.

The average particle diameter $D_{A1}$ of the inorganic particles (A1) are, from the viewpoint of improving the feel after application, 1 $\mu$m or more, preferably 1.5 $\mu$m or more, more preferably 2 $\mu$m or more, and is 50 $\mu$m or less, preferably 40 $\mu$m or less, more preferably 30 $\mu$m or less, even more preferably 25 $\mu$m or less, further more preferably 20 $\mu$m or less, further more preferably 15 $\mu$m or less, further more preferably 10 $\mu$m or less. More specifically, the average particle diameter $D_{A1}$ is, from the same viewpoint as above, preferably 1 to 40 $\mu$m, more preferably 1 to 30 $\mu$m, even more preferably 1 to 25 $\mu$m, further more preferably 1 to 20 $\mu$m, further more preferably 1 to 15 $\mu$m, further more preferably 1 to 10 $\mu$m, further more preferably 1.5 to 10 $\mu$m, further more preferably 2 to 10 $\mu$m.

The average particle diameter $D_{A1}$ is measured according to the method described in the section of Examples.

The surface roughness Ra of the inorganic particles (A1) is, from the viewpoint of improving the adhesion suppressing effect, 50 nm or less, preferably 40 nm or less, more preferably 35 nm or less, even more preferably 30 nm or less, and is 10 nm or more, preferably 12 nm or more, more preferably 14 nm or more, even more preferably 15 nm or more. More specifically, the surface roughness Ra is, from the same viewpoint as above, preferably 10 to 40 nm, more preferably 10 to 35 nm, even more preferably 12 to 35 nm, further more preferably 14 to 35 nm, further more preferably 15 to 35 nm, further more preferably 15 to 30 nm.

The surface roughness Ra is measured according to the method described in the section of Examples.

The inorganic particles (A1) are secondary particles, and the average secondary particle diameter thereof is the above-mentioned average particle diameter $D_{A1}$.

The average primary particle diameter $d_{A1}$ of the inorganic particles (A1) is 7 nm or more and 15 nm or less. The average primary particle diameter $d_{A1}$ is measured according to the method described in the section of Examples.

The surface roughness Ra is correlated with the average primary particle diameter $d_{A1}$ of the inorganic particles (A1), and accordingly, by controlling the average primary particle diameter $d_{A1}$, the surface roughness Ra can be controlled.

The inorganic particles (A1) are easily disintegrable secondary inorganic particles, from the viewpoint of improving the adhesion suppressing effect and improving the feel after rubbing.

In the present invention "disintegrable" is meant to indicate disintegrability of inorganic particles when rubbed under a predetermined load given thereto. Specifically, particles are rubbed for ten rounds of reciprocation using a surface property tester Tribogear Type 14 (by Shinto Scientific Co., Ltd.) according to the method stipulated in the section of Examples, and the particle diameter ratio thereof before and after rubbing represented by the following formula (I) is calculated, and the particles whose particle diameter ratio is less than 50% are referred to as "easily disintegrable particles", and the particles whose particle diameter ratio is 50% or more shown in the following formula (I) are referred to as "non-disintegrable particles".

$$\text{Particle Diameter Ratio before and after rubbing (\%)} = (\text{average particle diameter } D_{A1}\text{' after rubbing/ average particle diameter } D_{A1} \text{ before rubbing}) \times 100 \qquad \text{(I)}$$

The inorganic particles (A1) are easily disintegrable secondary particles, and therefore, when a coating film formed on the surface of a solid by applying the particle-containing composition onto the solid surface is rubbed, the inorganic particles (A1) are disintegrated into fine particles. With that, owing to the hydrogen-bonding interaction between the polar surfaces of the finely-pulverized inorganic particles (A1) and the amide bond or the amide group of the polymer (C) existing on the surfaces of the particles (B), the inorganic particles (A1) adhere to the surfaces of the particles (B) to form composite particles. It is considered that the composite particles are so configured that the finely-pulverized inorganic particles (A1) have adsorbed to the surfaces of the particles (B) serving as core particles, and the inorganic particles (A1) contribute toward forming nano-size surface irregularities to express the adhesion suppressing effect after rubbing. In addition, it is considered that, since the particles (B) constituting the composite particles have a predetermined average particle diameter, the composition can express the feel improving effect after rubbing. From this viewpoint, the particle diameter ratio that is an index of disintegrability is preferably 45% or less, more preferably 35% or less, even more preferably 25% or less.

The oil absorption amount of the inorganic particles (A1) is preferably 50 mL/100 g or more, more preferably 100 mL/100 g or more, even more preferably 130 mL/100 g or more, and is preferably 700 mL/100 g or less, more preferably 600 mL/100 g or less, even more preferably 500 mL/100 g or less. The oil absorption amount can be measured according to JIS K 5101-13-2:2004.

The inorganic particles (A1) that are prepared can be used, or commercial products can also be used.

In the case of preparing the inorganic particles (A1), a known method can be employed in accordance with the kind of the inorganic substance to constitute the particles (A1). For example, the particles can be prepared according to a high heat method such as a flame hydrolysis method or an arc method; or a wet method such as a sedimentation method or a gelling method. From the viewpoint of controlling the primary particle diameter of the inorganic particles (A1) to enhance the adhesion suppressing effect, the particles are preferably prepared by a gelling method. The gelling method can be carried out, for example, by emulsifying sodium silicate in an apolar organic solvent containing a surfactant to give a W/O emulsion and then gelling it. The average particle diameter $D_{A1}$ of the silica particles can be controlled by controlling the W/O emulsion size.

Preferably, the gelation is carried out by neutralization with a mineral acid such as sulfuric acid in an acidic region. In that manner, while suppressing the growth of the silica primary particles, the primary particles can aggregate to form silica secondary particles.

Next, in the aging step after gelation, the pore structure including the specific surface area, the pore diameter and the pore volume is controlled to control the primary particle diameter and the aggregation performance of the primary particles to thereby control the surface roughness Ra and the disintegrability of the resultant particles.

Commercial products of the inorganic particles (A1) include the silica particles such as Sunsphere H series (trade name by AGC SI-Tec Co., Ltd.).

The specific surface area of the silica particles is, from the viewpoint of enhancing the adhesion suppressing effect and bettering the feel after application, and also from the viewpoint of maintaining the excellent adhesion suppressing effect and the good feel after rubbing, preferably 100 m²/g or more, more preferably 300 m²/g or more, even more preferably 500 m²/g or more, and is preferably 1,000 m²/g or less, more preferably 900 m²/g or less, even more preferably 800 m²/g or less. The specific surface area is a BET specific surface area measured according to JIS Z 8830:2013.

The pore diameter of the silica particles is, from the viewpoint of improving the adhesion suppressing effect and improving the feel after application, and also from the viewpoint of maintaining the excellent adhesion suppressing effect and the good feel after rubbing, preferably 1 nm or more, more preferably 3 nm or more, even more preferably 5 nm or more, and is preferably 20 nm or less, more preferably 17 nm or less, even more preferably 15 nm or less.

The pore volume of the silica particles is, from the viewpoint of improving the adhesion suppressing effect and improving the feel after application, and also from the viewpoint of maintaining the excellent adhesion suppressing effect and the good feel after rubbing, preferably 0.3 mL/g or more, more preferably 0.5 mL/g or more, even more preferably 0.7 mL/g or more, and is preferably 3 mL/g or less, more preferably 2.7 mL/g or less, even more preferably 2.3 mL/g or less.

The pore diameter and the pore volume can be measured according to a gas adsorption method.

[Inorganic Particles (A2)]

The inorganic particles (A2) are inorganic particles having an average particle diameter $D_{A2}$ of less than 1 $\mu$m, and having an average primary particle diameter $d_{A2}$ of 10 nm or more and 20 nm or less.

The average particle diameter $D_{A2}$ of the inorganic particles (A2) is less than 1 $\mu$m, preferably 0.7 $\mu$m or less, more preferably 0.5 $\mu$m or less, even more preferably 0.3 $\mu$m or less.

The average primary particle diameter $d_{A2}$ of the inorganic particles (A2) is, from the viewpoint of improving the adhesion suppressing effect, 10 nm or more and 20 nm or less. More specifically, the average primary particle diameter $d_{A2}$ is, from the same viewpoint as above, 10 to 20 nm.

The inorganic particles (A2) may be any of primary particles or secondary particles.

The average primary particle diameter $d_{A2}$ can be determined on the observation image with a transmission electron microscope (TEM). Specifically, the particles are observed with TEM under the condition of an observation magnification power of 50,000 times, the maximum minor diameters of 300 primary particles on the observation image are measured, and the data are averaged to give a number-average value. Here, the maximum minor diameter means a minor diameter having a maximum length of minor diameters perpendicular to major diameter in the case where the inorganic particles (A2) have a shape different from a tabular one. In the case where the inorganic particles (A2) are tabular ones, the thicknesses of 300 primary particles in the image observed under the same condition as above are measured, and the data are averaged to give a number-average value.

Specifically, average particle diameter $D_{A2}$ and the average primary particle diameter $d_{A2}$ can be measured according to the method described in the section of Examples.

The inorganic particles (A2) that are prepared can be used, or commercial products can also be used.

In the case of preparing the inorganic particles (A2), a known method such as a vapor-phase method or a liquid-phase method can be employed in accordance with the kind of the inorganic substance to constitute the particles (A2). Above all, a vapor-phase method is preferred.

Regarding the vapor-phase method, for example, in the case where the inorganic particles (A2) are fine particles containing silicon dioxide (silica), the vapor-phase method for the particles includes a method of hydrolyzing a vapor of silicon tetrachloride at a high temperature to synthesize them, and a method of vaporizing and oxidizing a raw material of a metal powder in flame at a high temperature to synthesize fine particles of silica.

In the case where the inorganic particles (A2) are fine particles of silica that contain silica (hereinafter also referred to as "fine silica particles"), from the viewpoint of controlling the average particle diameter of the inorganic particles (A2) to enhance the adhesion suppressing effect, preferably employed is a flame hydrolysis method of forming silicon dioxide by processing silicon tetrachloride in a oxyhydrogen gas. By controlling the temperature for the flame hydrolysis treatment, the oxygen/hydrogen supply ratio, the supply amount of the raw material silicon tetrachloride, the residence time in flame and the zone length, the average particle diameter of the resultant particles can be controlled. Example of the flame hydrolysis treatment include a method described in JP 47-46274 B.

In the case where the inorganic particles (A2) are fine silica particles, the specific surface area of the fine silica particles is, from the viewpoint of enhancing the adhesion suppressing effect and bettering the feel after application, and also from the viewpoint of maintaining the excellent adhesion suppressing effect and the good feel after rubbing, preferably 50 $m^2$/g or more, more preferably, 100 $m^2$/g or more, even more preferably 150 $m^2$/g or more, and is preferably 400 $m^2$/g or less, more preferably 300 or less, even more preferably 250 or less. The specific surface area is a BET specific surface area measured according to JIS Z 8830:2013.

The fine silica particles of the inorganic particles (A2) can be hydrophilic fine silica particles, and can also be hydrophobic fine silica particles surface-treated with an alkylsilane. The surface treatment agent for hydrophobization includes polydimethylsiloxane, methylchlorosilane, dimethyldichlorosilane and hexamethyldisilazane. Above all, from the viewpoint of forming composite particles to improve the adhesion suppressing effect and to improve the feel in rubbing, preferred are hydrophilic fine silica particles.

The fine silica particles may contain any inorganic element than Si, such as Al, but the silica ($SiO_2$) content in the fine silica particles is preferably 90% by mass or more, more preferably 95% by mass or more, even more preferably 99% by mass or more, and is preferably 100% by mass or less.

Commercial products of fine silica particles include AEROSIL R972, AEROSIL R200, and AEROSIL R300 (all trade names by Evonik Corporation).

The content of the inorganic particles (A) in the particle-containing composition is, from the viewpoint of improving the adhesion suppressing effect and improving the feel after application, and also from the viewpoint of maintaining the excellent adhesion suppressing effect and the good feel after rubbing, preferably 3% by mass or more, more preferably 5% by mass or more, even more preferably 7% by mass or more, and is preferably 97% by mass or less, more preferably 95% by mass or less, even more preferably 90% by mass or less. More specifically, the content of the particles (A) in the particle-containing composition is preferably 3 to 97% by mass, more preferably 5 to 95% by mass, even more preferably 7 to 90% by mass.

The content of the inorganic particles (A1) in the particle-containing composition is, from the viewpoint of improving the adhesion suppressing effect and improving the feel after application, and also from the viewpoint of maintaining the excellent adhesion suppressing effect and the good feel after rubbing, preferably 5% by mass or more, more preferably 10% by mass or more, even more preferably 20% by mass or more, further more preferably 30% by mass or more, further more preferably 40% by mass or more, and is preferably 97% by mass or less, more preferably 95% by mass or less, even more preferably 90% by mass or less. More specifically, the content of the particles (A1) in the particle-containing composition is preferably 5 to 97% by mass, more preferably 10 to 95% by mass, even more preferably 20 to 90% by mass, further more preferably 30 to 90% by mass, further more preferably 40 to 90% by mass.

The content of the inorganic particles (A2) in the particle-containing composition is, from the viewpoint of improving the adhesion suppressing effect and improving the feel after application, and also from the viewpoint of maintaining the excellent adhesion suppressing effect and the good feel after rubbing, preferably 3% by mass or more, more preferably 5% by mass or more, even more preferably 7% by mass or more, and is preferably 90% by mass or less, more preferably 70% by mass or less, even more preferably 50% by mass or less, further more preferably 30% by mass or less. More specifically, the content of the particles (A2) in the particle-containing composition is preferably 3 to 90% by mass, more preferably 3 to 70% by mass, even more preferably 3 to 50% by mass, further more preferably 3 to 30% by mass, further more preferably 5 to 30% by mass, further more preferably 7 to 30% by mass.

<Particles (B)>

The particle-containing composition of the present invention contains particles (B) other than the inorganic particles (A), from the viewpoint of improving the feel after application and from the viewpoint of maintaining the good feel after rubbing. The particles (B) are particles having an average particle diameter $D_B$ of 1 $\mu$m or more and 50 $\mu$m or less, and coated on the surfaces thereof with a polymer (C) having an amide bond or an amide group in the side chain.

The average particle diameter $D_B$ of the particles (B) is, from the same viewpoint as above, preferably 1.3 $\mu$m or more, more preferably 1.5 $\mu$m or more, and is preferably 40 $\mu$m or less, more preferably 30 $\mu$m or less, even more preferably 25 $\mu$m or less, further more preferably 20 $\mu$m or less, further more preferably 15 $\mu$m or less, further more preferably 10 $\mu$m or less, further more preferably 7 $\mu$m or less. More specifically, the average particle diameter $D_B$ of the particles (B) is preferably 1 to 40 $\mu$m, more preferably 1 to 30 $\mu$m, even more preferably 1 $\mu$m to 25 $\mu$m, further more preferably 1 to 20 $\mu$m, further more preferably 1 to 15 $\mu$m, further more preferably 1 to 10 $\mu$m, further more preferably 1 to 7 $\mu$m, further more preferably 1.3 to 7 $\mu$m, further more preferably 1.5 to 7 $\mu$m.

The particles (B) are not specifically limited so far as coated with a polymer (C) having a an amide bond or an amide group in the side chain, but are preferably particles formed by coating the surfaces of at least one kind of particles (B') selected from the group consisting of inorganic particles and organic particles with the polymer (C).

In the case where the particles (B') are inorganic particles, the inorganic substance to constitute the inorganic particles may be selected from the group consisting of a silicon-containing compound such as silicon dioxide (silica), silicon nitride, silicon carbide, aluminum silicate, magnesium silicate, and calcium silicate; a metal oxide such as titanium oxide, zinc oxide, aluminum oxide (alumina), magnesium oxide, calcium oxide, zirconium oxide, iron oxide, cerium oxide, and chromium oxide; a metal salt such as magnesium carbonate, calcium carbonate, barium sulfate, barium carbonate, magnesium hydrogencarbonate, calcium hydrogencarbonate, lithium carbonate, calcium phosphate, titanium phosphate, silver chloride, and silver bromide; a metal hydroxide such as magnesium hydroxide, aluminum hydroxide, and calcium hydroxide; a metal such as titanium, iron, chromium, nickel, gold, silver, platinum, copper, lead, and zinc, and a metal alloy thereof; diamond; a boron-containing compound such as boron nitride, and boron carbide; a metal carbide such as titanium carbide, tantalum carbide, and zirconium carbide; a metal nitride such as aluminum nitride, and titanium nitride; and a mixture of magnesium metasilicate aluminate (magnesium silicate aluminate). One kind alone or two or more kinds of these inorganic particles can be used either singly or as combined. Above all, preferred are particles containing at least one selected from the group consisting of a silicon-containing compound and a metal oxide; more preferred are particles containing a silicon-containing compound; and even more preferred are silica particles.

In the case where the particles (B') are organic particles, the organic particles preferably contain at least one selected from the group consisting of a natural polymer, a semisynthetic polymer and a synthetic polymer. Specifically, the organic particles include polymer particles of polysaccharides and derivatives thereof such as cellulose, starch and chitosan, and natural waxes such as carnauba wax, candelilla wax, montan wax, paraffin wax and microcrystalline wax;

addition polymerization polymers such as polyolefins e.g., polystyrene, poly(meth)acrylate, polyvinyl acetate, polyethylene, and polypropylene; condensation polymers such as polyamides, polyesters e.g. polylactic acid, and polyurethanes; and silicone powders. The polymer particles may be polymer particles having a crosslinked structure, or may also be polymer particles not having a crosslinked structure.

The particles (B') are, from the viewpoint of improving the feel after application, preferably particles containing at least one selected from the group consisting of polystyrene, poly(meth)acrylate, polyamide, polylactic acid, silicone powder, silica, cellulose, cellulose derivatives, starch and starch derivatives, more preferably particles containing at least one selected from the group consisting of polystyrene, poly(meth)acrylate, cellulose and cellulose derivatives, even more preferably particles containing at least one selected from the group consisting of polystyrene and poly(meth)acrylate.

In this description, "(meth)acrylate" means at least one selected from the group consisting of acrylate and methacrylate.

The polystyrene includes a homopolymer or a copolymer of a styrene-based monomer. The styrene-based monomer is preferably at least one selected from the group consisting of styrene, 2-methylstyrene, $\alpha$-methylstyrene, vinyltoluene, and divinylbenzene, more preferably at least one selected from the group consisting of styrene, 2-methylstyrene and $\alpha$-methylstyrene, even more preferably at least one selected from the group consisting of styrene and $\alpha$-methylstyrene, further more preferably styrene.

The poly(meth)acrylate includes a homopolymer and a copolymer of a (meth)acrylic monomer. The (meth)acrylic monomer includes a (meth)acrylic acid; and a (meth)acrylate such as a (meth)acrylate having a hydrocarbon group derived from an aliphatic alcohol having 1 or more and 18 or less carbon atoms. One kind alone or two or more kinds of (meth)acrylic monomers can be used either singly or as combined.

In this description, "(meth)acrylic acid" means at least one selected from the group consisting of acrylic acid and methacrylic acid.

The poly(meth)acrylate may also be a crosslinked structure-having poly(meth)acrylate that has been further copolymerized with a polyfunctional (meth)acrylate such as ethylene glycol di(meth)acrylate, propylene glycol di(meth)acrylate and hexanediol di(meth)acrylate, in addition to (meth)acrylic acid and (meth)acrylate.

Specific examples of the poly(meth)acrylate include a polymethyl methacrylate, an acrylic acid/methyl acrylate copolymer, a butyl acrylate/ethylene glycol dimethacrylate/sodium methacrylate copolymer, and a lauryl methacrylate/ethylene glycol dimethacrylate/sodium methacrylate copolymer.

The above-mentioned cellulose derivatives include hydroxy cellulose, hydroxyalkyl cellulose, carboxy cellulose, carboxyalkyl cellulose, alkyl cellulose, hydroxyalkylalkyl cellulose, and cationized cellulose.

[Polymer (C)]

The polymer (C) has a an amide bond or an amide group in the side chain from the viewpoint of improving the feel after application, and from the viewpoint of maintaining the excellent adhesion suppressing effect and the good feel after rubbing. In the present invention, "amide bond" means a bond of a carbonyl group with a nitrogen atom bonding thereto.

The polymer (C) is preferably a polymer having an amide bond or an amide group in the side chain, more preferably a vinyl-based polymer produced by addition polymerization of a vinyl-based monomer having an amide bond or an amide group. The vinyl-based monomer includes an N-vinyl compound such as N-vinylpyrrolidone, N-vinylacetamide, N-vinylformamide, and N-vinylcaprolactam; and a (meth)acrylamide-based monomer such as (meth)acrylamide, and N-alkyl(meth)acrylamide. Among these, preferred are at least one selected from the group consisting of poly(N-vinylpyrrolidone) and poly(meth)acrylamide; and more preferred is poly(N-vinylpyrrolidone).

In this description, "(meth)acrylamide" means at least one selected from the group consisting of acrylamide and methacrylamide.

The poly(N-vinylpyrrolidone) is preferably at least one selected from the group consisting of an N-vinylpyrrolidone homopolymer, and a copolymer of N-vinylpyrrolidone and any other monomer. The poly(N-vinylpyrrolidone) of the type includes a N-vinylpyrrolidone homopolymer; and a copolymer of N-vinylpyrrolidone and any other monomer, such as N-vinylpyrrolidone/vinyl acetate copolymer, and N-vinylpyrrolidone/vinyl acetate/vinyl propionate polymer. Examples of commercial products of poly(N-vinylpyrrolidone) include PVP K-25, PVP K-30, and PVP K-90 (all trade names by FUJIFILM Wako Chemicals Corporation); PVA-6450, and Akone M (both trade names by Osaka Organic Chemical Industry Ltd.); and Corydon VA64 (vinylpyrrolidone/vinyl acetate copolymer, trade name by BASF Japan Corporation).

The poly(meth)acrylamide is preferably at least one selected from the group consisting of an N-vinylpyrrolidone homopolymer and a copolymer of N-vinylpyrrolidone and any other monomer. The poly(meth)acrylamide of the type include a homopolymer of a (meth)acrylic monomer; and a copolymer of a (meth)acrylic monomer and any other monomer such as (meth)acrylate or (meth)acrylic acid, and a salt thereof. Examples of the poly(meth)acrylamide include an N-tert-butylacrylamide/N,N-dimethylacrylamide/N-[3-(dimethylamino)propyl]acrylamide/methoxypolyethylene glycol methacrylate copolymer described in JP 2005-162700 A.

The blending amount of the polymer (C) relative to the particles (B) is 7% by mass or more and 35% by mass or less.

[Production of Particles (B)]

(Step 1)

The particles (B) can be produced according to the following step 1 using particles (B') to constitute the particles (B). Preferred inorganic particles and organic particles to constitute the particles (B') are preferably the same as those for the particles (B) mentioned hereinabove.

Step 1: a step of mixing particles (B') and the polymer (C) to give the particles (B).

Preferably, the mixing in the step 1 is carried out in a dispersion medium. For example, using water as a dispersion medium, an aqueous solution of a polymer (C) is dropwise added to an aqueous dispersion of particles (B'), then centrifuged to remove the supernatant, and the resultant precipitate is collected and dried to give particles (B). The resultant precipitant is preferably purified from the viewpoint of removing the unadsorbed polymer (C). The purification treatment can be carried out by, for example, the method described in the section of Examples.

Commercial products of the particles (B') include poly(meth)acrylate particles such as particles described as "crosslinked (meth)acrylate-based resin particles" in JP 2006-8980 A, and GANZPEARL series by Aica Kogyo Co., Ltd.; and cellulose particles such as CELLULOBEADS D series by Daito Kasei Kogyo Co., Ltd.

The blending amount of the polymer (C) relative to the particles (B') is 7% by mass or more and 55% by mass or less.

(Step 1')

The particles (B) can also be produced according to a method including the following step 1'.

Step 1': a step of polymerizing a monomer (b) to constitute the particles (B) in the presence of a polymer (C) to give the particles (B).

The polymerization method in the step 1' includes a known polymerization method such as a dispersion polymerization method, a suspension polymerization method and an emulsion polymerization method. Above all, from the viewpoint of controlling a particle diameter, improving the adhesion suppressing effect and improving the feel in application to skin, a dispersion polymerization method is preferred.

In dispersion polymerization, a solution prepared by dissolving a monomer (b), a dispersion medium, a polymerization initiator, and as a dispersion stabilizer, a polymer (C) is polymerized in the form of a uniform solution in the initial stage of polymerization, then polymer nuclei are formed by the polymer being precipitated, and via the growth of the polymer nuclei, particles (B) coated with the polymer (C) are finally formed.

The dispersion medium is not specifically limited so far as it can dissolve the monomer (b), the polymerization initiator and the polymer (C) as a dispersion stabilizer, and does not dissolve the particles (B) to be formed. Examples of the dispersion medium include a lower alcohol such as ethanol.

The polymerization initiator is preferably one capable of dissolving in the dispersion medium, more preferably an oil-soluble radical polymerization initiator such as dimethyl 2,2'-azobis(2-methylpropionate).

The amount of the polymerization initiator to be used can be appropriately selected depending on the kind and the amount of the monomer (b), the kind of the polymerization initiator or the polymerization temperature, and in general, preferably 0.01% by mass or more and 10% by mass or less, more preferably 0.1% by mass or more and 5% by mass or less, on the basis of the total amount of the monomer (b).

The concentration of the monomer (b) in the reaction system is not specifically limited, but is, from the viewpoint of production efficiency and of suppressing aggregation, preferably 1% by mass or more and 60% by mass or less.

The polymerization temperature can be set depending on the kind of the dispersion medium and the polymerization initiator, and is preferably 60°C or higher and 100°C or lower, more preferably 70°C or higher and 90°C or lower.

The polymerization time is preferably 1 hour or more and 30 hours or less, more preferably 6 hours or more and 24 hours or less.

The content of the particles (B) in the particle-containing composition is, from the viewpoint of improving the adhesion suppressing effect and improving the feel after application, and also from the viewpoint of maintaining the excellent adhesion suppressing effect and the good feel after rubbing, preferably 3% by mass or more, more preferably 5% by mass or more, even more preferably 10% by mass or more, and is preferably 97% by mass or less, more preferably 95% by mass or less, even more preferably 93% by mass or less. More specifically, the content of the particles (B) in the particle-containing composition is preferably 3 to 97% by mass, more preferably 5 to 95% by mass, even more preferably 10 to 93% by mass.

In the particle-containing composition, the mass ratio of the particles (B) to the inorganic particles (A) [(B)/(A)] is, from the viewpoint of improving the adhesion suppressing effect and improving the feel after application, and also from the viewpoint of maintaining the excellent adhesion suppressing effect and the good feel after rubbing, 0.1 or more, and 11 or less. More specifically, the mass ratio [(B)/(A)] is, from the same viewpoint as above, 0.1 to 11.

The mass ratio of the particles (B) to the inorganic particles (A1) [(B)/(A1)] is, from the viewpoint of improving the adhesion suppressing effect and improving the feel after application, and also from the viewpoint of maintaining the excellent adhesion suppressing effect and the good feel after rubbing, 0.1 or more, and is preferably 15 or less, more preferably 13 or less, even more preferably 10 or less, more preferably 7 or less, further more preferably 5 or less, further more preferably 3 or less, further more preferably 2 or less. More specifically, the mass ratio [(B)/(A1)] is, from the same viewpoint as above, 0.1 to 15, preferably 0.1 to 13, more preferably 0.1 to 10, further more preferably 0.1 to 7, further more preferably 0.1 to 5, further more preferably 0.1 to 3, further more preferably 0.1 to 2.

The mass ratio of the particles (B) to the inorganic particles (A2) [(B)/(A2)] is, from the viewpoint of improving the adhesion suppressing effect and improving the feel after application, and also from the viewpoint of maintaining the excellent adhesion suppressing effect and the good feel after rubbing, 0.1 or more, preferably 1 or more, further more preferably 3 or more, further more preferably 5 or more, further more preferably 7 or more, further more preferably 9 or more, and is preferably 15 or less, more preferably 13 or less, even more preferably 11 or less. More specifically, the mass ratio [(B)/(A2)] is, from the same viewpoint as above, 0.1 to 15, preferably 1 to 15, more preferably 3 to 15, further more preferably 5 to 15, further more preferably 5 to 13, further more preferably 7 to 13, further more preferably 7 to 11, further more preferably 9 to 11.

From the viewpoint of improving the adhesion suppressing effect, the particle-containing composition of the present invention is such that the surface roughness Ra of the coating form to be formed by applying the composition to the surface of a solid is preferably 50 nm or less, more preferably 40 nm or less, even more preferably 35 nm or less, further more preferably 30 nm or less, and is preferably 10 nm or more, more preferably 12 nm or more, even more preferably 14 nm or more, further more preferably 15 nm or more. More specifically, the surface roughness Ra of the coating film is, from the viewpoint of enhancing the adhesion suppressing effect, preferably 10 to 50 nm, more preferably 10 to 40 nm, even more preferably 10 to 35 nm, further more preferably 12 to 35 nm, further more preferably 14 to 35 nm, further more preferably 15 to 35 nm, further more preferably 15 to 30 nm.

The surface roughness Ra of the coating film is measured according to the method described in the section of Examples.

[Production Method for Particle-Containing Composition]

The particle-containing composition of the present invention can be produced according to an ordinary method depending on the use form thereof, and can be produced by mixing the inorganic particles (A), the particles (B) and various optional additives in a known method. Above all, from the viewpoint of enhancing the adhesion suppressing effect and bettering the feel after application, and also from the viewpoint of maintaining the excellent adhesion suppressing effect and the good feel after rubbing, the composition is preferably produced according to a method including the above-mentioned step 1 or step 1' followed by the step 2 mentioned below; and from the viewpoint production easiness, more preferably, the composition is produced according to the method that includes the following step 2 after the step 1. In the step 2, the mixing method is not specifically limited.

Step 2: a step of mixing the particles (B) and the inorganic particles (A) to give a particle-containing composition.

The particle-containing composition of the present invention can be used directly as it is without being diluted with a solvent, but depending on the use form and the intended object thereof, the composition can also be used after appropriately blended with a solvent, an oily agent and additives that are generally used in cosmetic materials.

The use form of the particle-containing composition of the present invention is not specifically limited. The use form may be any form including mist, liquid, foam, paste and cream, but preferred is a liquid, paste or cream form, more preferred is a liquid form.

Additives can be blended in the composition unless the objects of the present invention are adversely affected by inclusion thereof. The additives include, except the inorganic particles (A) and the particles (B), a dye, an organic pigment, an inorganic pigment, a UV scattering agent, a UV absorbent, a fragrance, a beauty component, a pharmaceutical component, a pH regulator, a viscosity regulator, a moisturizer, an antioxidant, a germicide, and a preservative. One kind alone or two or more kinds of these may be used either singly or as combined.

[Method for Suppressing Adhesion of Air Harmful Substances]

The particle-containing composition of the present invention has an excellent adhesion suppressing effect against air harmful substances and an excellent feel after application and can maintain the excellent adhesion suppressing effect and a good feel after rubbing, and is therefore favorably used as an adhesion suppressing agent against air harmful substances for the surface of a solid that is an object targeted for adhesion suppression.

Specifically, it is preferable that the particle-containing composition is applied to the surface of a solid to suppress air harmful substances from adhering to the solid surface.

In the present invention, "solid surface" means an interface between a solid and air.

The solid may be any of a biological solid or a non-biological solid.

The biological solid includes human and animal skin, hair and nail.

The non-biological solid includes glass, earthenware, porcelain, enamelware, tiles, ceramics, wood, metal; synthetic resins such as polyethylene, polypropylene, melamine resin, polyamide resin, ABS resin, and FRP; natural fibers such as cotton, silk, and wool; and synthetic fibers such as polyester, nylon and rayon.

The shape of the solid surface is not specifically limited.

The method of applying the particle-containing composition to a solid surface can be appropriately selected depending on the shape of the solid surface. In the case where the particle-containing composition is liquid, foam, paste or cream, in general, it can be applied directly as it is by coating or spraying.

In the adhesion suppressing method of the present invention, preferably, the composition is applied in such a manner that the surface roughness Ra of the coating film to be formed by applying the particle-containing composition to a solid surface can be 50 nm or less as mentioned above. The preferred range of the surface roughness Ra of the coating film in the method is the same as the preferred range of the surface roughness Ra of the above-mentioned coating film.

The coating amount of the particle-containing composition in applying the composition to a solid surface is, from the viewpoint of improving the adhesion suppressing effect, as the adhering amount of the inorganic particles (A), preferably 0.005 $mg/cm^2$ or more, more preferably 0.01 $mg/cm^2$ or more, and is, from the viewpoint of appearance and feel, preferably 5 $mg/cm^2$ or less, more preferably 3 $mg/cm^2$ or less, even more preferably 1 $mg/cm^2$ or less, further more preferably 0.8 $mg/cm^2$ or less. More specifically, the coating amount of the particle-containing composition in applying to a solid surface is, from the viewpoint of improving the adhesion suppressing effect and from the viewpoint of appearance and feel, as the adhering amount of the inorganic particles (A), preferably 0.005 to 5 $mg/cm^2$, more preferably 0.01 to 3 $mg/cm^2$, even more preferably 0.01 to 1 $mg/cm^2$, further more preferably 0.01 to 0.8 $mg/cm^2$.

The coating amount of the particle-containing composition in applying the composition to a solid surface is, from the viewpoint of improving the adhesion suppressing effect, as the adhering amount of the inorganic particles (A1), preferably 0.005 $mg/cm^2$ or more, more preferably 0.01 $mg/cm^2$ or more, even more preferably 0.03 $mg/cm^2$ or more, and is, from the viewpoint of appearance and feel, preferably 5 $mg/cm^2$ or less, more preferably 3 $mg/cm^2$ or less, even more preferably 1 $mg/cm^2$ or less, further more preferably 0.8 $mg/cm^2$ or less. More specifically, the coating amount of the particle-containing composition in applying to a solid surface is, from the viewpoint of improving the adhesion suppressing effect and from the viewpoint of appearance and feel, as the adhering amount of the inorganic particles (A1), preferably 0.005 to 5 $mg/cm^2$, more preferably 0.01 to 3 $mg/cm^2$, even more preferably 0.03 to 1 $mg/cm^2$, further more preferably 0.03 to 0.8 $mg/cm^2$.

The coating amount of the particle-containing composition in applying the composition to a solid surface is, from the viewpoint of improving the adhesion suppressing effect, as the adhering amount of the inorganic particles (A2), preferably 0.005 $mg/cm^2$ or more, more preferably 0.01 $mg/cm^2$ or more, and is, from the viewpoint of appearance and feel, preferably 5 $mg/cm^2$ or less, more preferably 3 $mg/cm^2$ or less, even more preferably 1 $mg/cm^2$ or less, further more preferably 0.8 $mg/cm^2$ or less. More specifically, the coating amount of the particle-containing composition in applying to a solid surface is, from the viewpoint of improving the adhesion suppressing effect and from the viewpoint of appearance and feel, as the adhering amount of the inorganic particles (A2), preferably 0.005 to 5 $mg/cm^2$, more preferably 0.005 to 3 $mg/cm^2$, even more preferably 0.01 to 3 $mg/cm^2$, further more preferably 0.01 to 1 $mg/cm^2$, further more preferably 0.01 to 0.8 $mg/cm^2$.

The particle-containing composition of the present invention has a high adhesion suppressing effect against air harmful substances and is excellent in the feel after application and further can maintain the excellent adhesion suppressing effect and the good feel after rubbing, and is therefore favorably applied to the surface of skin as a solid surface that is an object targeted for adhesion suppression. Specifically, it is preferable that the particle-containing composition is applied to skin to suppress air harmful substances from adhering to the skin. In applying to skin, the composition can be used as a material for imparting an adhesion suppressing effect against air harmful substances to skin external preparations or skin cosmetic materials, and above all, the composition is favorably used as a skin cosmetic material.

In the case where the particle-containing composition is liquid, foam, paste or cream, in general, it can be applied directly as it is by coating or spraying. Here, "apply to the surface of skin" is meant to include not only directly applying the particle-containing composition to the surface of skin by hand, but also adhering the particle-containing composition to the surface of skin by spraying.

The coating amount of the particle-containing composition in applying the composition to the surface of skin is, from the viewpoint of improving the adhesion suppressing effect, as the adhering amount of the inorganic particles (A), preferably 0.005 $mg/cm^2$ or more, more preferably 0.01 $mg/cm^2$ or more, and is, from the viewpoint of appearance and feel, preferably 5 mg/cm$^2$ or less, more preferably 3 mg/cm$^2$ or less, even more preferably 1 mg/cm$^2$ or less, further more preferably 0.8 mg/cm$^2$ or less. More specifically, the coating amount of the particle-containing composition in applying to the surface of skin is, from the viewpoint of improving the adhesion suppressing effect and from the viewpoint of appearance and feel, as the adhering amount of the inorganic particles (A), preferably 0.005 to 5 mg/cm$^2$, more preferably 0.01 to 3 mg/cm$^2$, even more preferably 0.01 to 1 mg/cm$^2$, further more preferably 0.01 to 0.8 mg/cm$^2$.

The coating amount of the particle-containing composition in applying the composition to the surface of skin is, from the viewpoint of improving the adhesion suppressing effect, as the adhering amount of the inorganic particles (A1), preferably 0.005 mg/cm$^2$ or more, more preferably 0.01 mg/cm$^2$ or more, even more preferably 0.03 mg/cm$^2$ or more, and is, from the viewpoint of appearance and feel, preferably 5 mg/cm$^2$ or less, more preferably 3 mg/cm$^2$ or less, even more preferably 1 mg/cm$^2$ or less, further more preferably 0.8 mg/cm$^2$ or less. More specifically, the coating amount of the particle-containing composition in applying to the surface of skin is, from the viewpoint of improving the adhesion suppressing effect and from the viewpoint of appearance and feel, as the adhering amount of the inorganic particles (A1), preferably 0.005 to 5 mg/cm$^2$, more preferably 0.01 to 3 mg/cm$^2$, even more preferably 0.03 to 1 mg/cm$^2$, further more preferably 0.03 to 0.8 mg/cm$^2$.

The coating amount of the particle-containing composition in applying the composition to the surface of skin is, from the viewpoint of improving the adhesion suppressing effect, as the adhering amount of the inorganic particles (A2), preferably 0.005 mg/cm$^2$ or more, more preferably 0.01 mg/cm$^2$ or more, and is, from the viewpoint of appearance and feel, preferably 5 mg/cm$^2$ or less, more preferably 3 mg/cm$^2$ or less, even more preferably 1 mg/cm$^2$ or less, further more preferably 0.8 mg/cm$^2$ or less. More specifically, the coating amount of the particle-containing composition in applying to the surface of skin is, from the viewpoint of improving the adhesion suppressing effect and from the viewpoint of appearance and feel, as the adhering amount of the inorganic particles (A2), preferably 0.005 to 5 mg/cm$^2$, more preferably 0.005 to 3 mg/cm$^2$, even more preferably 0.01 to 3 mg/cm$^2$, further more preferably 0.01 to 1 mg/cm$^2$, further more preferably 0.01 to 0.8 mg/cm$^2$.

Regarding the above-mentioned embodiments, the present invention further discloses the following embodiments.

<1> A particle-containing composition that contains inorganic particles (A) and particles (B) other than the particles (A), wherein:

the inorganic particles (A) are at least one selected from the group consisting of the following inorganic particles (A1) and inorganic particles (A2),
the particles (B) are particles having an average particle diameter $D_B$ of 1 $\mu$m or more and 50 $\mu$m or less, and coated on the surfaces thereof with a polymer (C) having an amide bond or an amide group in the side chain,

Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less,

Inorganic particles (A2): inorganic particles having an average particle diameter $D_{A2}$ of less than 1 $\mu$m, and having an average primary particle diameter $d_{A2}$ of 10 nm or more and 20 nm or less,

the inorganic particles (A) are particles containing silica,
the mass ratio of particles (B) to inorganic particles (A), [(B)/(A)], is 0.1 or more and 11 or less, and
the blending amount of the polymer (C) relative to the particles (B) is 3% by mass or more and 50% by mass or less.

<2> A method for producing the particle-containing composition, wherein the particles (B) are particles prepared by coating the surfaces of particles (B') with the polymer (C), the method including the following step 1 and step 2,

Step 1: a step of mixing particles (B') and the polymer (C) to give the particles (B),
Step 2: a step of mixing the particles (B) obtained in the step 1 with the inorganic particles (A) to give a particle-containing composition.

<3> A method for suppressing adhesion of air harmful substances, including applying the particle-containing composition to the surface of a solid in an amount, as the coating amount of the inorganic particles (A), of 0.005 mg/cm$^2$ or more and 5 mg/cm$^2$ or less to suppress air harmful substances from adhering to the solid surface.

EXAMPLES

In the following Examples and Comparative Examples, "%" is "% by mass", unless otherwise specified.

Various properties were measured according to the following methods.

(1) Measurement of average particle diameter $D_{A1}$, $D_{A2}$ and $D_B$

Measured using a laser diffraction/scattering particle size distribution measurement apparatus (trade name "LA-920", by Horiba, Ltd.), and using ionexchanged water as a dispersion medium. In measuring organic particles, the relative refractive index was 1.10, and in measuring inorganic particles, the relative refractive index was 1.09. For the measurement, a flow cell was used. Regarding the measurement conditions, the circulation rate was 1.5 with no ultrasonic irradiation, the distribution form was standard, and the particle diameter was on a volume basis.

(2) Measurement of average primary particle diameter $d_{A1}$ and $d_{A2}$

In the case where the measurement sample has any other shape than a tabular one, a dispersion of the measurement sample prepared previously was put on a sample stage of a transmission electron microscope (TEM) (trade name "JEM1400Plus" by JEOL Corporation), air-dried thereon, and the maximum minor diameter of each of 300 primary particles on the image taken by TEM at an observation magnification of 50,000x was measured, and the resultant data were averaged to give a number-average value to be the average primary particle diameter of the sample. Here, the maximum minor diameter means a minor diameter having a maximum length of minor diameters perpendicular to the major diameter.

In the case where the measurement sample is a tabular one, the thickness of each of 300 primary particles was measured on the image taken according to the same method and under the same observation magnification condition, and the resultant data were averaged to give a number-average value to be the average primary particle diameter of the sample.

A dispersion of the measurement sample was prepared by ultrasonically dispersing 5 g of the measurement sample in 95 g of ethanol added thereto as a solvent.

(3) Measurement of surface roughness Ra of particles

A dispersion of particles (dispersion medium: ethanol, solid concentration: 5%) was uniformly applied onto an artificial leather (trade name, "Laforet S2923" by Okamoto Shinwa Co., Ltd.) in an amount of 2.0 mg/cm$^2$ (amount of adhered particles: 0.1 mg/cm$^2$), taking 20 seconds, and dried at room temperature for 24 hours to give measurement samples. Next, the surface of the measurement sample coated with particles was analyzed using a scanning probe microscope system (scanning probe microscope unit: AFM5100N, scanning probe station: AFM5000II) (by Hitachi High-Tech Corporation) under measurement conditions of cantilever: SI-DF20P2, PM mode, scanning range: 20 $\mu$m $\times$ 20 $\mu$m, correction: secondary curve), and the linear roughness Ra at 500 nm thus measured was referred to as a surface roughness Ra.

(4) Confirmation of disintegrability

0.04 g of a dispersion of particles (dispersion medium: ethanol, solid concentration: 5%) was uniformly applied and spread on an artificial leather (Laforet S2923) (5 cm $\times$ 4 cm) taking 20 seconds (amount of adhered particles: 0.1 mg/cm$^2$), and dried at room temperature for 24 hours to give measurement samples. Using a surface property tester (trade name "Tribogear Type: 14", by Shinto Scientific Co., Ltd.) (vertical load: 360 g, moving distance: 50 mm, moving speed: 600 mm/min), the particles-coated surface of the measurement sample was rubbed for 10 reciprocations with a tool (3 cm $\times$ 3 cm) having an uncoated artificial leather (Laforet S2923) (3 cm $\times$ 10 cm) attached thereto. Next, using a field emission scanning electron microscope (FE-SEM) (Model: 4800 (5.0 kV), by Hitachi High-Tech Corporation), the rubbed surface of the measurement sample was observed at an observation magnification of 15,000x. The major diameter and the minor diameter of each of 30 particles on the observation image were measured, and an average value of the major diameter and the minor diameter was referred to as the particle diameter of each articles. From the found data of the particle diameter of 30 particles, a volume-based particle diameter was calculated to be the average particle diameter $D_{A1}'$ after rubbing.

With reference to the average particle diameter $D_{A1}'$ after rubbing and the average particle diameter $D_{A1}$ before rubbing as measured in the above (1), particles having a particle diameter ratio represented by the following formula (I) of less than 50% were referred to as "easily disintegrable particles".

Particle Diameter Ratio before and after rubbing (%) = (average particle diameter $D_{A1}'$ after rubbing/ average particle diameter $D_{A1}$ before rubbing) $\times$ 100     (I)

(5) Measurement of surface roughness Ra of coating film of particle-containing composition

A dispersion of each particle-containing composition (dispersion medium: ethanol, solid concentration: 5%) was uniformly applied onto an artificial leather (Laforet S2923) in an amount of 2.0 mg/cm$^2$ (amount of adhered particle-containing composition: 0.1 mg/cm$^2$), taking 20 seconds, and dried at room temperature for 24 hours to give measurement samples. Next, the surface of the measurement sample coated with the particle-containing composition was analyzed, using a scanning probe microscope system (scanning probe microscope unit: AFM5100N, scanning probe station: AFM5000II) (by Hitachi High-Tech Corporation) under measurement conditions of cantilever: SI-DF20P2, PM mode, scanning range: 20 $\mu$m $\times$ 20 $\mu$m, correction: secondary curve), and the linear roughness Ra at 500 nm thus measured was referred to as a surface roughness Ra.

The inorganic particles (A) used in Examples and Comparative Examples are as follows.

The average particle diameter $D_A$, the average primary particle diameter $d_A$ and the surface roughness Ra of the inorganic particles (A), and the disintegrability of the particles are shown in Table 1.

[Inorganic Particles (A1)]

Silica particles A1-1: trade name "Sunsphere H-32", by AGC SI-Tec Co., Ltd., oil absorption amount 300 mL/100 g, specific surface area 700 m$^2$/g, pore diameter 10 nm, pore volume 2 mL/g

[Inorganic Particles (A2)]

Fine silica particles A2-1: trade name "AEROSIL 200", by Nippon Aerosil Co., Ltd., specific surface area 200 $\pm$ 25

Production Example 1 (polymethacrylate particles B'2)

According to Example 1 in JP 2006-8980 A, polymethacrylate particles B'2 (particles of lauryl methacrylate/ethylene glycol dimethacrylate/sodium methacrylate copolymer) were synthesized.

Example 1

(Step 1')

14.4 g of a polymer (C), polyvinylpyrrolidone (trade name "PVP K-30", by FUJIFILM Wako Chemicals Corporation) (hereinafter also referred to as "PVP"), 100.2 g of a monomer (b), styrene monomer (by FUJIFILM Wako Chemicals Corporation), 1.0 g of a polymerization initiator, V-601 (2,2'-azobis(2-methylpropionate), trade name by FUJIFILM Wako Chemicals Corporation), and 684.9 g of ethanol (first class grade chemical, by FUJIFILM Wako Chemicals Corporation) were put into a 1-L separable flask, and dissolved by stirring. The resultant solution was heated up to 80°C with stirring by a mechanical stirrer (150 rpm) in an oil bath for dispersion polymerization (polymerization time: 20 h). The resultant reaction liquid was spontaneously cooled, and then centrifuged with a centrifuge (trade name "CR21GIII", by Koki Holdings Co., Ltd.) (treatment condition: 5,000 rpm, 15 min). The supernatant was removed, and ethanol (800 mL) was added to the resultant precipitate for redispersion with an ultrawasher (trade name "UT-205", by Sharp Corporation) (15 min). Using the above centrifuge, this was centrifuged (treatment condition: 5,000 rpm, 15 min) and the supernatant was removed to give a precipitate for purification treatment. The purification treatment was repeated for a total of 3 times, and after drying, white PVP-coated polystyrene particles B1 were obtained.

(Step 2)

The PVP-coated polystyrene particles B1 obtained in the step 1' and silica particles A1-1 were mixed with a stirrer in the mass ratio [(B)/(A)] shown in Table 1 to give an ethanol dispersion of particle-containing composition (content of particle-containing composition: 5%)

Example 2

(Step 1)

10 g of the polymethacrylate particles B'2 obtained in Production Example 1, and 190 g of ethanol were put into a 300-mL plastic container, and stirred with a stirrer (600 rpm). 100 g of an ethanol solution of a polymer (C), polyvinyl pyrrolidone (PVP K-30) (concentration 1%) was dropwise added to the resultant dispersion of polymethacrylate particles B'2, taking 30 minutes, and kept stirred for 1 hour after the dropwise addition. Subsequently, this was centrifuged with a centrifuge (CR21GIII) (treatment condition; 10,000 rpm, 15 min). The supernatant was removed, and ethanol (200 mL) was added to the resultant precipitate. After redispersion using an ultrasonic washer (UT-205) (15 min), this was again centrifuged (1,000 rpm, 15 min) using the above centrifuge for purification treatment to give a precipitate. The purification treatment was repeated for a total of 3 times, then the unadsorbed polymer (C) was removed, and after drying, PVP-coated polymethacrylate particles B2-1 were obtained.

(Step 2)

The PVP-coated polymethacrylate particles B2-1 obtained in the step 1 and silica particles A1-1 were mixed with a stirrer in the mass ratio [(B)/(A)] shown in Table 1 to give an ethanol dispersion of particle-containing composition (content of particle-containing composition: 5%)

Example 3

An ethanol dispersion of particle-containing composition (content of particle-containing composition: 5%) was obtained according to the same method as above, except that, in the step 1 in Example 2, cellulose particles B'3 (trade name "CELLULOBEADS D-5", by Daito Kasei Kogyo Co., Ltd.) were used in place of the polymethacrylate particles B'2.

Examples 4, 5

An ethanol dispersion of particle-containing composition (content of particle-containing composition: 5%) was obtained according to the same method as above, except that, in Example 2, the blending amount of the polymer (C) relative to the particles (B) and the mass ratio [(B)/(A)] were changed as in Table 1.

Example 6

An ethanol dispersion of particle-containing composition (content of particle-containing composition: 5%) was obtained according to the same method as above, except that, in the step 2 in Example 2, fine silica particles A2-1 were used in place of the silica particles A1-1 and the mass ratio [(B)/(A)] was changed as in Table 1.

Comparative Example 1

The polymethacrylate particles B'2 obtained in Production Example 1 and the silica particles A1-1 were mixed using a stirrer in the mass ratio [(B)/(A)] as in Table 1 to give an ethanol dispersion of particle-containing composition (content of particle-containing composition: 5%).

Comparative Example 2

Not using the particles (B), an ethanol dispersion of the silica particles A1-1 alone (content of silica particles A1-1: 5%) was prepared.

[Evaluation]

The ethanol dispersions of Examples and Comparative Examples were evaluated according to the following methods. As Reference Example 1, a skin or an artificial leather as a substitute for skin, which had not been coated, was evaluated in the same manner.

<Evaluation of Adhesion Suppression>

The ethanol dispersion of particle-containing composition (in Comparative Example 2, the ethanol dispersion of silica particles A1-1 alone) was applied to a white artificial leather (Laforet S2923) as a substitute for skin to have a coating amount of 0.1 mg/cm$^2$ as a solid content, and dried at room temperature for 24 hours.
The surface of the artificial leather coated with the particle-containing composition was exposed to an air flow environment of air harmful substances for evaluation, and using a colorimeter, the L*a*b* value was measured, and a color difference ΔE before and after exposure was measured according to the following method to evaluate adhesion suppression. The results are shown in Table 2.

[Measurement of color difference ΔE]

Using a colorimeter (trade name "NF 777" by Nippon Denshoku Industries Co., Ltd.), the $L_1^*$, $a_1^*$ and $b_1^*$ values of the surface of the artificial leather coated with the particle-containing composition, before exposed to air harmful substances for evaluation were measured.

Separately, a fan (trade name "Silky Wind 9ZF002RH02", size: 129 × 106 × 83 mm, by Rhythm Co., Ltd.) and a wire sieve (test sieve by JIS Z 8801, frame dimension: φ100 × 45 H, opening: 106 μm, by Tokyo Screen Co., Ltd.) were fixed in a glove bag (part number "3-118-01", by AS ONE Corporation). The installation height of the wire sieve was 17 cm.

The artificial leather (5 cm × 4 cm) coated with the particle-containing composition of the test sample was attached to a support so that the height of the lower end of the artificial leather was 11 cm. The distance between the coated surface of the artificial leather on the support and the fan was 15 cm, and as shown in Fig. 1, the coated surface of the artificial leather was set to be perpendicular to the blowing direction of the fan, and the height of the center of the blade of the fan was to be the same as the height of the center of the artificial leather.

The temperature in the glove bag was 25°C and the relative humidity therein was 57% RH. Using a brush for removing clogging from the wire sieve (trade name "JNB-5", opening: 106 μm or less, brush diameter 53 μm, by Tokyo Screen Co., Ltd.), 0.05 g of air harmful substances for evaluation shown in Table 2 were, while classified, dropped down for 1 minute before the blowout port of the fan whose blowout grade was set at 1. In that manner, the surface of the artificial leather coated with the particle-containing composition was exposed to the air flow environment of air harmful substances for evaluation.

Next, using the above colorimeter, the $L_2^*$, $a_2^*$, $b_2^*$ values of the exposed surface of the artificial leather were measured, and the color difference ΔE value was calculated according to the following formula (II).

$$\Delta E = [(L_1^* \text{-} L_2^*)^2 + (a_1^* \text{-} a_2^*)^2 + (b_1^* \text{-} b_2^*)^2]^{0.5} \qquad \text{(II)}$$

The color difference ΔE of the artificial leather coated with the particle-containing composition of the test sample was referred to as ΔEt, the color difference ΔE of the artificial leather not coated with the particle-containing composition was referred to as ΔEs, and from the ratio of ΔEt to ΔEs (ΔEt/ΔEs) (N = 3), the adhesion suppressing effect was evaluated. A ratio (ΔEt/ΔEs) of less than 1.0 means that the sample has an adhesion suppressing effect against air harmful substances. A smaller ratio (ΔEt/ΔEs) indicates a more excellent adhesion suppressing effect.

The air harmful substances for evaluation shown in Table 2 are as follows.

Graphite powder: trade name "J-CPB", average particle diameter: 5.5 μm, by Nippon Graphite Industries, Ltd.

<Feel (dry silky feel) Evaluation>

Four expert panelists tried external preparations by applying 0.02 mL of the ethanol dispersion of particle-containing composition (content of particle-containing component: 5%) to the inner portion of the forearm to be in a circular form having a diameter of 3 cm, followed by uniformly spreading it thereover under the condition of 25°C and 57% RH, taking 20 seconds. The panelists made five times of organoleptic evaluation for the feel (dry silky feel) after ethanol evaporation according to the following criteria. An average score of the four panelists was calculated. The results are shown in Table 2.

    5: Much dry silky feel.
    4: Dry silky feel.
    3: No specific feel.
    2: Squeaky feel.
    1: Much squeaky feel.

Further, the ethanol dispersion of particle-containing composition obtained in Examples was evaluated in point of adhesion suppression and feel after rubbing, according to the following method.

Since the samples of Comparative Examples 1 and 2 were poor in any of the adhesion suppressing effect and the feel improving effect evaluated in the above, these were not evaluated any more in point of the following adhesion suppression after rubbing. The results are shown in Table 2.

<Evaluation of adhesion suppression after rubbing>

0.04 g of the ethanol dispersion of particle-containing composition (content of particle-containing composition:

5%) was uniformly applied and spread on an artificial leather (Laforet S2923) (5 cm × 4 cm) taking 20 seconds, and dried at room temperature for 24 hours to give measurement samples of particle-containing composition-coated artificial leather. Using a surface property tester, Tribogear Type: 14 (by Shinto Scientific Co., Ltd.) (vertical load: 360 g, moving distance: 50 mm, moving speed: 600 mm/min), the surface of each particle-containing composition-coated test sample was rubbed for 10 reciprocations with a tool (3 cm × 3 cm) having an uncoated artificial leather (Laforet S2923) (3 cm × 10 cm) attached thereto.

The rubbed test samples were collected and evaluated for adhesion suppression according to the same method as above. The results are shown in Table 2.

<Feel Evaluation after rubbing>

Four expert panelists tried external preparations by applying 0.02 mL of the ethanol dispersion of particle-containing composition (content of particle-containing component: 5%) to the inner portion of the forearm to be in a circular form having a diameter of 3 cm, followed by uniformly spreading it thereover under the condition of 25°C and 57% RH, taking 20 seconds. After ethanol evaporation, the panelists rubbed the coated portion with hand for a total of 10 times, and then made five times of organoleptic evaluation for the feel (dry silky feel) after rubbing according to the following criteria. An average score of the four panelists was calculated. The results are shown in Table 2.

     5: Much dry silky feel.
     4: Dry silky feel.
     3: No specific feel.
     2: Squeaky feel.
     1: Much squeaky feel.

Table 1

| | | | | Particle-Containing Composition | | | | | | | | | | | | | |
|---|---|---|---|---|---|---|---|---|---|---|---|---|---|---|---|---|---|
| | | | Inorganic Particles (A) | | | | | | | | | Particles (B) | | | | Mass Ratio [(B)/(A)] | Surface Roughness of Coating Film of Particle-Containing Composition (nm) |
| | | Kind | | Average Particle Diameter $D_{A1}$ (μm) | Average Primary Particle Diameter $d_{A1}$ (nm) | Average Particle Diameter $D_{A2}$ (μm) | Average Primary Particle Diameter $d_{A2}$ (nm) | Surface Roughness Ra (nm) | Particle Diameter Ratio (%) *1 | Disintegrability | Content in Particle-Containing Composition (mass%) | Kind | Average Particle Diameter $D_B$ (μm) | Polymer (C) | | | |
| | | | | | | | | | | | | | | Kind | Blending Amount (mass%) *2 | | |
| Example | 1 | Inorganic Particles (A1) | Silica Particles A1-1 | 3 | 10 | - | - | 23 | 20 | Easily disintegrable | 50 | PVP-Coated Polystyrene Particles B1 | 2 | PVP | 13 | 1.0 | 21 |
| | 2 | | Silica Particles A1-1 | 3 | 10 | - | - | 23 | 20 | Easily disintegrable | 50 | PVP-Coated Polymethacrylate Particles B2-1 | 3 | PVP | 9 | 1.0 | 25 |
| | 3 | | Silica Particles A1-1 | 3 | 10 | - | - | 23 | 20 | Easily disintegrable | 50 | PVP-Coated Cellulose Particles B3 | 5 | PVP | 9 | 1.0 | 24 |
| | 4 | | Silica Particles A1-1 | 3 | 10 | - | - | 23 | 20 | Easily disintegrable | 67 | PVP-Coated Polymethacrylate Particles B2-2 | 3 | PVP | 14 | 0.5 | 23 |
| | 5 | | Silica Particles A1-1 | 3 | 10 | - | - | 23 | 20 | Easily disintegrable | 83 | PVP-Coated Polymethacrylate Particles B2-3 | 3 | PVP | 33 | 0.2 | 22 |
| | 6 | Inorganic Particles (A2) | Silica Fine Particles A2-1 | - | - | 0.1 | 12 | - | - | - | 9 | PVP-Coated Polymethacrylate Particles B2-1 | 3 | PVP | 9 | 10 | 25 |
| Reference Example | 1 | - | - | - | - | - | - | - | - | - | - | | | | | - | - |

(continued)

## Particle-Containing Composition

| | | Inorganic Particles (A1) Kind | Inorganic Particles (A) | | | | | | | | Particles (B) | | Polymer (C) | | Mass Ratio [(B)/(A)] | Surface Roughness of Coating Film of Particle-Containing Composition (nm) |
|---|---|---|---|---|---|---|---|---|---|---|---|---|---|---|---|---|
| | | | Average Particle Diameter $D_{A1}$ (μm) | Average Primary Particle Diameter $d_{A1}$ (nm) | Average Particle Diameter $D_{A2}$ (μm) | Average Primary Particle Diameter $d_{A2}$ (nm) | Surface Roughness Ra (nm) | Particle Diameter Ratio (%) *1 | Disintegrability | Content in Particle-Containing Composition (mass%) | Kind | Average Particle Diameter $D_B$ (μm) | Kind | Blending Amount (mass%) *2 | | |
| Comparative Example | 1 | Silica Particles A1-1 | 3 | 10 | - | - | 23 | 20 | Easily disintegrable | 50 | Polymethacrylate Particles B'2 | 3 | - | 0 | 1.0 | 8 |
| | 2 | Silica Particles A1-1 | 3 | 10 | - | - | 23 | 20 | Easily disintegrable | 100 | - | - | - | - | 0 | 23 |

*1: Particle diameter ratio (%) = (average particle diameter $D_{A1}$ after rubbing/average particle diameter $D_{A1}$ before rubbing) × 100 *2: Blending amount (mass%) of polymer (C) relative to particles (B)

Table 2

| | | Evaluation | | | | | | |
|---|---|---|---|---|---|---|---|---|
| | | Blending Amount of Particle-Containing Composition (mass%)*1 | Air Harmful Substances for Evaluation | Adhering Amount of Inorganic Particles (A) (mg/cm$^2$) | Before rubbing | | After rubbing | |
| | | | | | Adhesion Suppressing Effect | Feel | Adhesion Suppressing Effect | Feel |
| Example | 1 | 5 | Graphite powder | 0.05 | 0.6 | 4.2 | 0.3 | 4.0 |
| | 2 | 5 | Graphite powder | 0.05 | 0.5 | 4.1 | 0.3 | 4.1 |
| | 3 | 5 | Graphite powder | 0.05 | 0.6 | 3.5 | 0.4 | 2.7 |
| | 4 | 5 | Graphite powder | 0.07 | 0.5 | 3.1 | 0.3 | 3.0 |
| | 5 | 5 | Graphite powder | 0.08 | 0.3 | 3.7 | 0.2 | 3.6 |
| | 6 | 5 | Graphite powder | 0.01 | 0.4 | 3.4 | 0.2 | 3.2 |
| Reference Example | 1 | - | Graphite powder | - | 1.0 | 3.0 | - | - |
| Comparative Example | 1 | 5 | Graphite powder | 0.05 | 1.4 | 3.3 | - | - |
| | 2 | 5 | Graphite powder | 0.10 | 0.2 | 2.8 | - | - |
| *1: Blending amount (mass%) of particle-containing composition in dispersion of particle-containing composition | | | | | | | | |

From Table 2, it is known that, as compared with the uncoated state in Reference Example 1, the particle-containing compositions of Examples 1 to 6 have a high adhesion suppressing effect and are excellent in feel and, in addition, can maintain the high adhesion suppressing effect and the good feel even after rubbing.

On the other hand, it is known that, in Comparative Example 1, the polymethacrylate particles B'2 are not coated with the polymer (C) and therefore cannot express an adhesion suppressing effect.

It is known that, in Comparative Example 2, the composition contains the inorganic particles (A) alone, not containing the particles (B), and therefore can have an adhesion suppressing effect but is poor in feel.

Industrial Applicability

The particle-containing composition of the present invention has a high adhesion suppressing effect and is excellent in feel and, in addition, can maintain the high adhesion suppressing effect and the good feel even after rubbing, and is therefore especially useful as an adhesion suppressing agent against air harmful substances that has adhesion suppressing performance against air harmful substances for use by applying to the surface of a solid.

Reference Signs List

1:    Sample Targeted for Evaluation
2:    Support
3:    Wire Sieve
4:    Air Harmful Substances for Evaluation

5: Fan

**Claims**

1. A particle-containing composition that comprises inorganic particles (A) and particles (B) other than the particles (A), wherein:

   the inorganic particles (A) are at least one selected from the group consisting of the following inorganic particles (A1) and inorganic particles (A2),
   the particles (B) are particles having an average particle diameter $D_B$ of 1 $\mu$m or more and 50 $\mu$m or less, and coated on the surfaces thereof with a polymer (C) having an amide bond or an amide group in the side chain,
   Inorganic particles (A1): easily disintegrable secondary inorganic particles having an average particle diameter $D_{A1}$ of 1 $\mu$m or more and 50 $\mu$m or less, having a surface roughness Ra of 10 nm or more and 50 nm or less, and having an average primary particle diameter $d_{A1}$ of 7 nm or more and 15 nm or less,
   Inorganic particles (A2): inorganic particles having an average particle diameter $D_{A2}$ of less than 1 $\mu$m, and having an average primary particle diameter $d_{A2}$ of 10 nm or more and 20 nm or less,
   the inorganic particles (A) are particles containing silica,
   the mass ratio of particles (B) to inorganic particles (A), [(B)/(A)], is 0.1 or more and 11 or less, and
   the blending amount of the polymer (C) relative to the particles (B) is 7% by mass or more and 35% by mass or less.

2. The particle-containing composition according to claim 1, wherein the particles (B) are particles formed by coating the surfaces of organic particles comprising at least one selected from the group consisting of a natural polymer, a semisynthetic polymer and a synthetic polymer, preferably the group consisting of polystyrene, poly(meth)acrylate, cellulose and cellulose derivatives, with the polymer (C).

3. The particle-containing composition according to claim 1 or 2, wherein the polymer (C) comprises a vinyl-based polymer produced by addition polymerization of a vinyl monomer having an amide bond or an amide group, preferably the vinyl monomer is at least one monomer selected from the group consisting of an N-vinyl compound and a (meth)acrylamide-based monomer, and preferably the polymer (C) is at least one selected from the group consisting of a poly(N-vinylpyrrolidone) and a poly(meth)acrylamide.

4. The particle-containing composition according to any one of claims 1 to 3, wherein the surface roughness Ra of the coating film formed by applying the particle-containing composition to the surface of a solid is 50 nm or less.

5. The particle-containing composition according to any one of claims 1 to 4, wherein the content of the inorganic particles (A) in the particle-containing composition is 3% by mass or more and 97% by mass or less.

6. The particle-containing composition according to any one of claims 1 to 5, which is used as a skin cosmetic material.

7. A method for producing a particle-containing composition of any one of claims 1 to 6 wherein the particles (B) are particles prepared by coating the surfaces of particles (B') with the polymer (C), the method comprising the following step 1 and step 2,

   Step 1: a step of mixing particles (B') and the polymer (C) to give the particles (B),
   Step 2: a step of mixing the particles (B) obtained in the step 1 with the inorganic particles (A) to give a particle-containing composition.

8. A method for suppressing adhesion of air harmful substances, comprising applying a particle-containing composition of any one of claims 1 to 6 to the surface of a solid to suppress adhesion of air harmful substances to the solid surface.

9. The method for suppressing adhesion of air harmful substances according to claim 8, wherein the surface roughness Ra of the coating film formed by applying the particle-containing composition to the surface of a solid is 50 nm or less.

10. The method for suppressing adhesion of air harmful substances according to claim 8 or 9, wherein the solid surface is a skin surface.

11. The method for suppressing adhesion of air harmful substances according to any one of claims 8 to 11, wherein the particle-containing composition is applied to the solid surface in an amount, as the adhering amount of the inorganic particles (A), of 0.005 mg/cm$^2$ or more and 5 mg/cm$^2$ or less.

**Patentansprüche**

1. Partikelhaltige Zusammensetzung, umfassend anorganische Partikel (A) und Partikel (B), die von den Partikeln (A) verschieden sind, wobei:

   die anorganischen Partikel (A) mindestens eines, ausgewählt aus der Gruppe, bestehend aus den folgenden anorganischen Partikel (A1) und anorganischen Partikel (A2), sind,
   die Partikel (B) Partikel mit einem durchschnittlichen Partikeldurchmesser $D_B$ von 1 μm oder mehr und 50 μm oder weniger sind und auf ihren Oberflächen mit einem Polymer (C), das eine Amidbindung oder eine Amidgruppe in der Seitenkette aufweist, beschichtet sind,
   Anorganische Partikel (A1): leicht desintegrierbare sekundäre anorganische Partikel mit einem durchschnittlichen Partikeldurchmesser $D_{A1}$ von 1 μm oder mehr und 50 μm oder weniger, die eine Oberflächenrauheit Ra von 10 nm oder mehr und 50 nm oder weniger aufweisen und einen durchschnittlichen Primärpartikeldurchmesser $d_{A1}$ von 7 nm oder mehr und 15 nm oder weniger aufweisen,
   Anorganische Partikel (A2): anorganische Partikel mit einem durchschnittlichen Partikeldurchmesser $D_{A2}$ von weniger als 1 μm und mit einem durchschnittlichen Primärpartikeldurchmesser $d_{A2}$ von 10 nm oder mehr und 20 nm oder weniger,
   die anorganischen Partikel (A) siliciumoxidhaltige Partikel sind,
   das Massenverhältnis von Partikeln (B) zu anorganischen Partikeln (A), [(B)/(A)], 0,1 oder mehr und 11 oder weniger beträgt, und
   die Mischungsmenge des Polymers (C), bezogen auf die Partikel (B), 7 Massen-% oder mehr und 35 Massen-% oder weniger beträgt.

2. Partikelhaltige Zusammensetzung gemäß Anspruch 1, wobei die Partikel (B) Partikel sind, die durch Beschichten der Oberflächen von organischen Partikeln, umfassend mindestens eines, ausgewählt aus der Gruppe, bestehend aus einem natürlichen Polymer, einem halbsynthetischen Polymer und einem synthetischen Polymer, bevorzugt aus der Gruppe, bestehend aus Polystyrol, Poly(meth)acrylat, Cellulose und Cellulosederivaten, mit dem Polymer (C) gebildet werden.

3. Partikelhaltige Zusammensetzung gemäß Anspruch 1 oder 2, wobei das Polymer (C) ein vinylbasiertes Polymer umfasst, das durch Additionspolymerisation eines Vinylmonomers mit einer Amidbindung oder einer Amidgruppe hergestellt wird, wobei das Vinylmonomer bevorzugt mindestens ein Monomer ist, das aus der Gruppe ausgewählt ist, die aus einer N-Vinylverbindung und einem (Meth)acrylamid-basierten Monomer besteht, und wobei das Polymer (C) bevorzugt mindestens eines ist, das aus der Gruppe ausgewählt ist, die aus einem Poly(N-vinylpyrrolidon) und einem Poly(meth)acrylamid besteht.

4. Partikelhaltige Zusammensetzung gemäß einem der Ansprüche 1 bis 3, wobei die Oberflächenrauheit Ra des Beschichtungsfilms, der durch Aufbringen der partikelhaltigen Zusammensetzung auf die Oberfläche eines Festkörpers gebildet wird, 50 nm oder weniger beträgt.

5. Partikelhaltige Zusammensetzung gemäß einem der Ansprüche 1 bis 4, wobei der Gehalt der anorganischen Partikel (A) in der partikelhaltigen Zusammensetzung 3 Massen-% oder mehr und 97 Massen-% oder weniger beträgt.

6. Partikelhaltige Zusammensetzung gemäß einem der Ansprüche 1 bis 5, die als hautkosmetisches Material verwendet wird.

7. Verfahren zur Herstellung einer partikelhaltigen Zusammensetzung gemäß einem der Ansprüche 1 bis 6, wobei die Partikel (B) Partikel sind, die durch Beschichten der Oberflächen von Partikeln (B') mit dem Polymer (C) hergestellt werden, wobei das Verfahren die folgenden Schritte 1 und 2 umfasst,

   Schritt 1: einen Schritt des Mischens von Partikeln (B') und dem Polymer (C), um die Partikel (B) zu erhalten,
   Schritt 2: einen Schritt des Mischens der in Schritt 1 erhaltenen Partikel (B) mit den anorganischen Partikeln (A), um eine partikelhaltige Zusammensetzung zu erhalten.

8. Verfahren zur Unterdrückung der Adhäsion von Luftschadstoffen, umfassend das Aufbringen einer partikelhaltigen Zusammensetzung gemäß einem der Ansprüche 1 bis 6 auf die Oberfläche eines Festkörpers, um die Adhäsion von Luftschadstoffen an der Festkörperoberfläche zu unterdrücken.

9. Verfahren zur Unterdrückung der Adhäsion von Luftschadstoffen gemäß Anspruch 8, wobei die Oberflächenrauheit Ra des Beschichtungsfilms, der durch Aufbringen der partikelhaltigen Zusammensetzung auf die Oberfläche eines Festkörpers gebildet wird, 50 nm oder weniger beträgt.

10. Verfahren zur Unterdrückung der Adhäsion von Luftschadstoffen gemäß Anspruch 8 oder 9, wobei die Festkörperoberfläche eine Hautoberfläche ist.

11. Verfahren zur Unterdrückung der Adhäsion von Luftschadstoffen gemäß einem der Ansprüche 8 bis 11, wobei die partikelhaltige Zusammensetzung in einer Menge als anhaftende Menge der anorganischen Partikel (A) von 0,005 mg/cm$^2$ oder mehr und 5 mg/cm$^2$ oder weniger auf die Festkörperoberfläche aufgebracht wird.

**Revendications**

1. Composition contenant des particules qui comprend des particules inorganiques (A) et des particules (B) autres que les particules (A), dans laquelle :

   les particules inorganiques (A) sont au moins une particule sélectionnée parmi le groupe constitué des particules inorganiques (A1) et des particules inorganiques (A2) suivantes,
   les particules (B) sont des particules présentant un diamètre moyen de particule $D_B$ de 1 μm ou plus et de 50 μm ou moins, et revêtues sur leurs surfaces avec un polymère (C) présentant une liaison amide ou un groupe amide dans la chaîne latérale,
   Particules inorganiques (A1) : particules inorganiques secondaires facilement désintégrables présentant un diamètre moyen de particule $D_{A1}$ de 1 μm ou plus et de 50 μm ou moins, présentant une rugosité de surface Ra de 10 nm ou plus et de 50 nm ou moins, et présentant un diamètre moyen de particule primaire $d_{A1}$ de 7 nm ou plus et de 15 nm ou moins,
   Particules inorganiques (A2) : particules inorganiques présentant un diamètre moyen de particule $D_{A2}$ inférieur à 1 μm, et présentant un diamètre moyen de particule primaire $d_{A2}$ de 10 nm ou plus et de 20 nm ou moins,
   les particules inorganiques (A) sont des particules contenant de la silice,
   le rapport de masse de particules (B) à des particules inorganiques (A), [(B)/(A)] est de 0,1 ou plus et de 11 ou moins, et
   la quantité de mélange du polymère (C) par rapport aux particules (B) est de 7 % en masse ou plus et de 35 % en masse ou moins.

2. Composition contenant des particules selon la revendication 1, dans laquelle les particules (B) sont des particules formées en revêtant les surfaces de particules organiques comprenant au moins un polymère sélectionné parmi le groupe consistant en un polymère naturel, un polymère semi-synthétique et un polymère synthétique, de préférence le groupe consistant en du polystyrène, du poly(méth)acrylate, de la cellulose et des dérivés de cellulose, avec le polymère (C).

3. Composition contenant des particules selon la revendication 1 ou la revendication 2,
   dans laquelle le polymère (C) comprend un polymère à base de vinyle produit par polymérisation par addition d'un monomère vinylique présentant une liaison amide ou un groupe amide, de préférence le monomère vinylique est au moins un monomère sélectionné parmi le groupe consistant en un composé N-vinylique et un monomère à base de (méth)acrylamide, et de préférence le polymère (C) est au moins un élément sélectionné parmi le groupe consistant en du poly(N-vinylpyrrolidone) et du poly(méth)acrylamide.

4. Composition contenant des particules selon l'une quelconque des revendications 1 à 3, dans laquelle la rugosité de surface Ra du film de revêtement formé en appliquant la composition contenant des particules sur la surface d'un solide est de 50 nm ou moins.

5. Composition contenant des particules selon l'une quelconque des revendications 1 à 4, dans laquelle la teneur en particules inorganiques (A) dans la composition contenant des particules est de 3 % en masse ou plus et de 97 % en masse ou moins.

6. Composition contenant des particules selon l'une quelconque des revendications 1 à 5, qui est utilisée comme matière cosmétique pour la peau.

7. Procédé de production d'une composition contenant des particules selon l'une quelconque des revendications 1 à 6, dans lequel les particules (B) sont des particules préparées en revêtant les surfaces de particules (B') avec le polymère (C), le procédé comprenant l'étape 1 et l'étape 2 suivantes,

   Étape 1 : une étape de mélange de particules (B') et du polymère (C) pour donner les particules (B),
   Étape 2 : une étape de mélange des particules (B) obtenues à l'étape 1 avec les particules inorganiques (A) pour donner une composition contenant des particules.

8. Procédé de suppression d'adhérence de polluant atmosphérique nocif, comprenant une application d'une composition contenant des particules selon l'une quelconque des revendications 1 à 6 sur la surface d'un solide pour supprimer l'adhérence de polluant atmosphérique nocif sur la surface solide.

9. Procédé de suppression d'adhérence de polluant atmosphérique nocif selon la revendication 8, dans lequel la rugosité de surface Ra du film de revêtement formé en appliquant la composition contenant des particules sur la surface d'un solide est de 50 nm ou moins.

10. Procédé de suppression d'adhérence de polluant atmosphérique nocif selon la revendication 8 ou la revendication 9, dans lequel la surface solide est une surface de peau.

11. Procédé de suppression d'adhérence de polluant atmosphérique nocif selon l'une quelconque des revendications 8 à 11, dans lequel la composition contenant des particules est appliquée sur la surface solide en une quantité, en tant que la quantité adhérente des particules inorganiques (A), de 0,005 mg/cm$^2$ ou plus et de 5 mg/cm$^2$ ou moins.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012250917 A
- WO 2014136993 A
- US 2005074474 A1
- US 4205997 A
- JP 47046274 B
- JP 2005162700 A
- JP 2006008980 A

**Non-patent literature cited in the description**

- **SHIRAIWA.** *Nature Chemistry,* 2011, vol. 3, 291-295
- GANZPEARL. Aica Kogyo Co., Ltd,
- CELLULOBEADS D. Daito Kasei Kogyo Co., Ltd